(19) Europäisches Patentamt
European Patent Office
Office européen des brevets

(11) **EP 4 628 571 A1**

(12) **EUROPEAN PATENT APPLICATION**
published in accordance with Art. 153(4) EPC

(43) Date of publication:
08.10.2025 Bulletin 2025/41

(21) Application number: 23897673.2

(22) Date of filing: 24.11.2023

(51) International Patent Classification (IPC):
*C12N 5/07* (2010.01)   *C12M 1/00* (2006.01)
*C12N 1/00* (2006.01)

(52) Cooperative Patent Classification (CPC):
C12N 5/0602; C12M 1/00; C12M 33/00;
C12M 33/08; C12M 41/48; C12M 47/04; C12N 1/00;
C12N 5/06; C12N 2509/10

(86) International application number:
PCT/JP2023/042136

(87) International publication number:
WO 2024/117025 (06.06.2024 Gazette 2024/23)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.11.2022 JP 2022190040

(71) Applicant: CANON KABUSHIKI KAISHA
Tokyo 146-8501 (JP)

(72) Inventors:
• SHIMOZAWA, Hideharu
  Tokyo 146-8501 (JP)
• FURUI, Takaaki
  Tokyo 146-8501 (JP)
• YAMAUCHI, Fumio
  Tokyo 146-8501 (JP)

(74) Representative: TBK
Bavariaring 4-6
80336 München (DE)

(54) **CELL DETACHMENT METHOD, CELL DETACHMENT SYSTEM, AND INFORMATION PROCESSING DEVICE**

(57)    A cell detachment method for detaching a cell adhering to a culture surface of a cell culture container includes an adhesion strength reduction step of reducing the adhesion strength of the cell by applying a stimulus to the cell while the cell remains adhered to the cell culture container, a measurement step of measuring the cell and acquiring cell information at at least two time points, a detachment step of detaching the cell from the culture surface, and a determination step of determining, based on the cell information, a time point t2 at which the detachment step is to be started, the time point t2 being after a time point t1, the time point t1 being a later time point among the at least two time points.

FIG. 9

```
         START

REDUCING CELL ADHESION        S101
STRENGTH TO CULTURE SURFACE

ACQUIRING CELL INFORMATION    S102

DETERMINING TIME POINT AT WHICH   S103
DETACHMENT STEP IS TO BE STARTED

DETACHING CELL                S104

          END
```

## Description

Technical Field

The present invention relates to a cell detachment method, a cell detachment system, and an information processing device.

Background Art

In recent years, there has been a major paradigm shift in drug development, from conventional low-molecular-weight drugs and antibody drugs to cell medicine, which utilizes cells themselves as drugs, and regenerative medicine, which aims to regenerate tissues and organs. In this cell medicine and regenerative medicine, a large number of cells are required, and in particular, it is required to efficiently and stably supply adherent cells that occupy most of the biological tissue.

In adherent cell culture, target cells are acquired through the steps of culturing cells on a culture substrate, such as a polystyrene dish, detaching the cells from the substrate, and recovering the cells, and washing the cells. When an increase in the number of cells is desired, what is called a subculture operation is performed in which some of the resulting cells are transferred to a new substrate and cultured. In this series of steps, the detachment step of detaching the cells from the substrate has been studied for efficient and stable supply of the cells.

Patent Literature 1 discloses that, in a device for sorting detached cells, the ratio of the projected areas of individual adherent cells before the start of detachment and during the detachment process is calculated and compared to a predetermined threshold value to determine whether the cells have been detached from the substrate.

Patent Literature 2 discloses that, in a device for determining cell detachment, brightness information is compared with a predetermined brightness level based on data obtained by photographing cells to determine whether the cells have been detached.

Patent Literature 3 discloses that, in a device for evaluating cell proliferation ability, individual anchorage-dependent cells are repeatedly photographed after the application of a detachment agent, and data on cell proliferation ability is displayed based on temporal changes in the projected areas of the cells.

Citation List

Patent Literature

PTL 1: Japanese Patent Laid-Open No. 2003-235540
PTL 2: International Publication No. 2007/136073
PTL 3: Japanese Patent Laid-Open No. 2004-344049

Summary of Invention

Technical Problem

The techniques described in Patent Literatures 1 to 3 disclose determining the end of the detachment operation and evaluating cell proliferation ability by measuring cell information. However, the Patent Literatures 1 to 3 do not disclose a method for determining a start time point of the detachment operation. The inventors have paid attention to the fact that the adhesion strength of cells varies in accordance with conditions, such as cell types and culture conditions. The inventors have conducted intensive studies and have found that in the case where a start time point is not set to an appropriate time point corresponding to the state of the cells to be detached and where a detachment operation is performed, the cell survival rate and the cell detachment efficiency obtained by the detachment step are sometimes reduced.

It is an object of the present invention to provide a cell detachment method that achieves a high cell survival rate and detachment efficiency.

Solution to Problem

A cell detachment method disclosed in this specification is a cell detachment method for detaching a cell adhering to a culture surface of a cell culture container, the method including an adhesion strength reduction step of reducing the adhesion strength of the cell by applying a stimulus to the cell while the cell remains adhered to the cell culture container, a measurement step of measuring the cell and acquiring cell information at at least two time points, a detachment step of detaching the cell from the culture surface, and a determination step of determining, based on the cell information, a time point $t2$ at which the detachment step is to be started, the time point $t2$ being after a time point $t1$, the time point $t1$ being a later time point among the at least two time points.

A cell detachment method disclosed in this specification is a cell detachment method for detaching a cell adhering to a culture surface of a cell culture container, the method including an adhesion strength reduction step of reducing the adhesion strength of the cell by applying a stimulus to the cell while the cell remains adhered to the cell culture container, a measurement step of measuring the cell and acquiring time-series data relating to the cell, a detachment step of detaching the cell from the culture surface, and a prediction step of predicting, based on the time-series data, a time point at which the detachment step is to be started.

A cell detachment system disclosed in this specification is a cell detachment system for detaching a cell adhering to a culture surface of a cell culture container, the system including an adhesion strength reduction unit to reduce the adhesion strength of the cell by applying a stimulus to the cell while the cell remains adhered to the cell culture container, a measurement unit to measure the cell and acquire cell information at at least two time points, a detachment unit to detach the cell from the culture surface; and a determination unit to determine, based on the cell information, a time point $t2$ at which a detachment step of detaching the cell from the culture surface is to be started, the time point $t2$ being after a time point $t1$, the time point $t1$ being a later time point among the at least two time points.

A cell detachment system disclosed in this specification is a cell detachment system for detaching a cell adhering to a culture surface of a cell culture container, the system including an adhesion strength reduction unit to reduce the adhesion strength of the cell by applying a stimulus to the cell while the cell remains adhered to the cell culture container, a measurement unit to measure the cell and acquire time-series data relating to the cell, a detachment unit to detach the cell from the culture surface, and, a prediction unit to predict, based on the time-series data, a time point at which a detachment step of detaching the cell from the culture surface is to be started.

An information processing device disclosed in this specification is an information processing device for controlling a cell detachment apparatus to detach a cell adhering to a culture surface of a cell culture container, the information processing device including a measurement unit to acquire, at at least two time points, the cell information of the cell having reduced adhesion strength to the culture surface due to the application of a stimulus, and a determination unit to determine, based on the cell information, a time point $t2$ at which the detachment step of detaching the cell from the culture surface is to be started, the time point $t2$ being after a time point $t1$, the time point $t1$ being a later time point among the at least two time points.

An information processing device disclosed in this specification is an information processing device for controlling a cell detachment apparatus to detach a cell adhering to a culture surface of a cell culture container, the information processing device including a measurement unit to acquire, at at least two time points, time-series data relating to the cell having reduced adhesion strength to the culture surface due to the application of a stimulus, and a prediction unit to predict, based on the time-series data, a time point at which a detachment step of detaching the cell from the culture surface is to be started. Advantageous Effects of Invention

According to the present invention, it is possible to provide the cell detachment method that achieves a high cell survival rate and detachment efficiency.

Brief Description of Drawings

[Fig. 1] Fig. 1 is a diagram illustrating a configuration of an information processing system capable of executing a program according to an embodiment of the present invention.

[Fig. 2] Fig. 2 is a schematic diagram illustrating a cell detachment apparatus according to an embodiment of the present invention.

[Fig. 3] Fig. 3 illustrates an example of measuring the area fraction of a halo region as cell information in an embodiment of the present invention.

[Fig. 4] Fig. 4 illustrates the measurement results of cell information and the results of curve fitting in Example 1 of the present invention.

[Fig. 5] Fig. 5 illustrates the measurement results of cell information and the results of curve fitting in Example 4 of the present invention.

[Fig. 6] Fig. 6 illustrates the measurement results of cell information and the results of curve fitting in Example 5 of the present invention.

[Fig. 7] Fig. 7 illustrates the measurement results of cell information and the results of curve fitting in Example 6 of the present invention.

[Fig. 8] Fig. 8 illustrates a shear stress application mechanism according to Example 7 of the present invention.

[Fig. 9] Fig. 9 is a flowchart illustrating a cell detachment method according to an embodiment of the present invention.
[Fig. 10] Fig. 10 is a flowchart illustrating a cell detachment method according to an embodiment of the present invention.
[Fig. 11] Fig. 11 illustrates a configuration of a cell detachment system according to an embodiment of the present invention.

Description of Embodiments

The present invention will be described in detail below with reference to preferred embodiments.

A cell detachment method disclosed in this specification is a cell detachment method for detaching a cell adhering to a culture surface of a cell culture container, the method including a measurement step of measuring the cell and acquiring cell information at at least two time points, a detachment step of detaching the cell from the culture surface, and a determination step of determining, based on the cell information, a time point t2 at which the detachment step is to be started, the time point t2 being after a time point t1, the time point t1 being a later time point among the at least two time points at which the cell information is measured.

A cell detachment method disclosed in this specification is a cell detachment method for detaching a cell adhering to a culture surface of a cell culture container, the method including an adhesion strength reduction step of reducing the adhesion strength of the cell by applying a stimulus to the cell while the cell remains adhered to the cell culture container, a measurement step of measuring the cell and acquiring time-series data relating to the cell, a detachment step of detaching the cell from the culture surface, and a prediction step of predicting, based on the time-series data, a time point at which the detachment step is to be started.

As a result of intensive studies on the detachment step by the inventors, it has been found that when the detachment operation is started before the adhesion strength of the cells is sufficiently reduced, the cell detachment efficiency and the cell survival rate are disadvantageously reduced. It has also been found that when a step of reducing the adhesion strength of cells is performed for a long period of time, the cell survival rate is disadvantageously reduced because the cells are placed in an environment different from the culture environment for a long period of time. As described above, the inventors have found that there is an appropriate start time point of cell detachment in the detachment step.

In addition, the inventors have found that a preliminary operation time up to a start time point of the detachment operation can be provided by measuring cell information at at least two time points and determining, based on the cell information, the start time point of the detachment operation after the time point at which the cell information is measured. The inventors have found that the presence of the preliminary operation time can provide a cell detachment method with a high degree of flexibility in device design. For example, as the cell detachment system, a configuration may be used in which a measurement device that measures cell information and a detachment apparatus that performs the detachment operation are separately provided, and the culture container is moved from the measurement device to the detachment apparatus during the preliminary operation time. In addition, by providing the preliminary operation time, a device that performs the detachment operation, for example, an ultrasonic irradiation device can be prepared to start the operation, and thus the detachment operation can be performed at an appropriate timing.

From the results of the above studies, the inventors have found that it is possible to provide a cell detachment method having a high cell survival rate and high detachment efficiency by providing a method for determining the start time point of the detachment operation after the time point of measuring the cell information in the cell detachment step.

(Cell Culture Container)

The substrate in the embodiment means a cell culture container used for cell culture. The cell culture container is not particularly limited as long as it is a culture container that is cellular adhesive. Examples of the cell culture container include flasks, tissue culture flasks, dishes, Petri dishes, tissue culture dishes, multi-dishes, microplates, multi-well plates, multi-plates, Petri dishes, culture bags, and bottles.

The material of the cell culture container in the embodiment is only required to be a material that is chemically stable and capable of culturing desired cells. The material of the cell culture container is, for example, at least one selected from the group consisting of polyethylene, polypropylene, polycarbonate, polystyrene, polyvinyl chloride, nylon, polyurethane, polyurea, polylactic acid, polyglycolic acid, polyvinyl alcohol, polyvinyl acetate, poly(meth)acrylic acid, poly(meth)acrylic acid derivatives, polyacrylonitrile, poly(meth)acrylamide, poly(meth)acrylamide derivatives, polysulfone, cellulose, cellulose derivatives, polysilicone, polymethylpentene, glass, and metal. Among them, polystyrene is preferable as the material of the cell culture container.

(Cell)

The cells in the embodiment are not particularly limited as long as they can adhere to and be cultured on a culture substrate in vitro. Examples thereof include various cultured cell lines, such as Chinese hamster ovary-derived CHO cells, mouse connective tissue L929 cells, mouse skeletal muscle myoblasts (C2C12 cells), human fetal lung-derived normal diploid fibroblasts (TIG-3 cells), human fetal kidney-derived cells (HEK293 cells), human alveolar basal epithelial adenocarcinoma-derived A549 cells, and human cervical cancer-derived HeLa cells. Further examples thereof include epithelial cells and endothelial cells constituting various tissues and organs in a living body, skeletal muscle cells, smooth muscle cells, and cardiac muscle cells, which exhibit contractility, neuronal cells, glial cells, and fibroblasts, which constitute the nervous system, hepatic parenchymal cells, hepatic non-parenchymal cells, and adipocytes, which are involved in the metabolism of the living body, and cells having differentiation potency, such as various stem cells, **e.g.,** induced pluripotent stem (iPS) cells, embryonic stem (ES) cells, embryonic reproductive (EG) cells, embryonic cancer (EC) cells, mesenchymal stem cells, hepatic stem cells, pancreatic stem cells, skin stem cells, muscle stem cells, and germ stem cells, progenitor cells of various tissues, and cells induced to differentiate from these cells. Among these, the cell detachment method according to the embodiment is suitable for cells having strong cell-to-cell bonding and cells having high adhesion strength to the culture surface of the cell culture container. Due to the need for mass culture of cells, the method is particularly suitable for, for example, CHO cells used for protein production and mesenchymal stem cells that can be used in cell therapy. The cells in the embodiment may be cell sheets cultured in a sheet form.

(Medium)

The type of the medium is not particularly limited. Examples thereof include Dulbecco's Modified Eagles's Medium (DMEM), Ham's F12 Medium (Ham's Nutrient Mixture F12), DMEM/F12 medium, McCoy's 5A medium, Eagles's Minimum Essential Medium (EMEM), alpha Modified Eagles's Minimum Essential Medium ($\alpha$MEM), Minimum Essential Medium (MEM medium), RPMI1640 medium, Iscove's Modified Dulbecco's Medium (IMDM), MCDB131 medium, William's medium E, IPL41 medium, Fischer's medium, StemSpan H3000 (manufactured by STEMCELL Technologies), StemSpan SFEM (manufactured by STEMCELL Technologies), StemlineII (manufactured by Sigma-Aldrich), Endothelial Cell Growth Medium 2 Kit (manufactured by PromoCell), Mesenchymal StemCell Growth Medium 2 (manufactured by PromoCell), MSCGM Bullet Kit (manufactured by Lonza), mTeSR1 or 2 medium (manufactured by STEMCELL Technologies), ReproFF or ReproFF2 (manufactured by ReproCELL), NutriStem medium (manufactured by Biological Industries), and MF-Medium mesenchymal stem cell growth medium (manufactured by Toyobo **Co.,** Ltd.).
Of these, a medium suitable for the culture of a target cell is preferably used.

(Serum)

To the above-described medium, serum or an antibiotic may be added. Examples of the serum that can be used include fetal bovine serum (FBS), calf serum, adult bovine serum, horse serum, sheep serum, goat serum, pig serum, chicken serum, rabbit serum, and human serum. In general, FBS is often used because of its easy availability. The medium may be a serum-free medium containing neither untreated nor unrefined serum and containing a component derived from serum or a compound derived from animal tissue (growth factor or the like).

(Antibiotic)

Examples of the antibiotic added to the medium include penicillin, streptomycin, ampicillin, carbenicillin, tetracycline, bleomycin, actinomycin, kanamycin, actinomycin D, and amphotericin **B.**

(Cell Culture Conditions)

Cell culture conditions can be appropriately selected in accordance with the cells to be cultured. Typically, an appropriate medium is added to a dish. Cells are seeded therein at about $1.0 \times 10^1$ to $5.0 \times 10^4$ cells/cm$^2$ and cultured in an environment at 37°C and a CO$_2$ concentration of 5%. In this case, the cells are preferably cultured until the proportion of the cell-occupied area in the substrate reaches about 70% to 80%, that is, the cells are in what is called a subconfluent state.

(Cell Detachment Method)

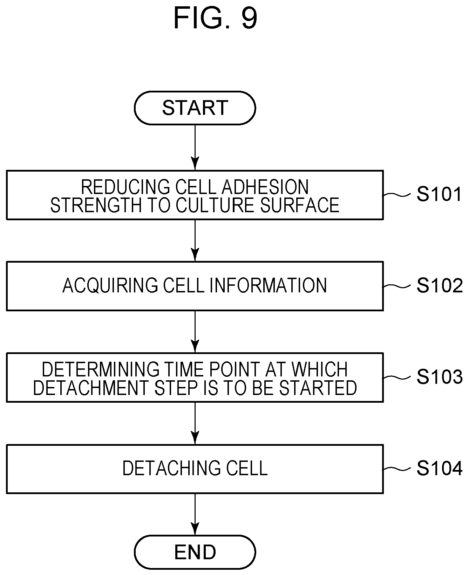

A cell detachment method according to an embodiment of the present invention is a cell detachment method for detaching cells adhering to a culture surface of a cell culture container. Fig. 9 is a flowchart illustrating a cell detachment method according to the embodiment. The cell detachment method includes an adhesion strength reduction step S101 of reducing the cell adhesion strength to a culture surface, a measurement step S102 of acquiring cell information, a determination step S103 of determining a time point t2 at which a detachment step is to be started, and the detachment step S104 of detaching cells from the culture surface.

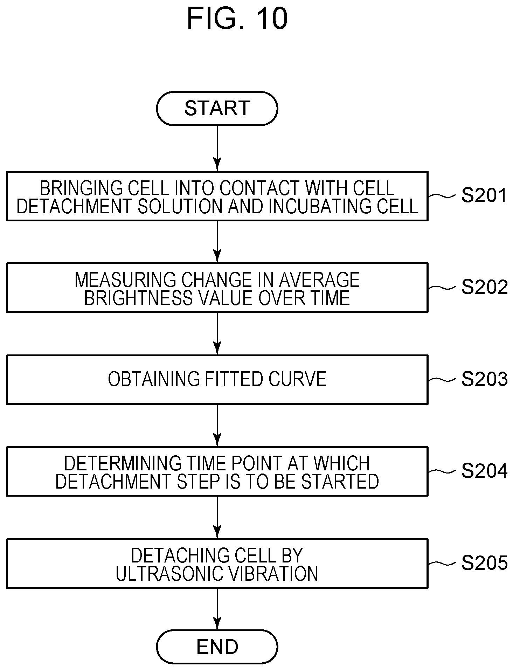

Fig. 10 illustrates a flowchart of another example of a cell detachment method according to an embodiment of the present invention. The other example of the cell detachment method includes an incubation step S201 of bringing cells into contact with a cell and incubating the cells, and a measurement step S202 of measuring a temporal change in the average brightness value of a cell image. The other example of the cell detachment method further includes a step S203 of obtaining a fitted curve from the average brightness value and the time point, a determination step S204 of determining the start time point of the detachment step, and a detachment step S205 of detaching the cells by ultrasonic vibration.

Each step will be described below.

(Adhesion Strength Reduction Step)

The adhesion strength reduction step according to an embodiment of the present invention is a step of reducing the adhesion strength of cells by applying a stimulus to the cell while the cell remains adhered to a substrate. Specific means to reduce the adhesion strength of cells include chemical stimuli, such as a cell detachment solution, mechanical stimuli, such as tapping and flow, electromagnetic stimuli, such as light, electricity, and magnetism, and thermal stimuli, such as heating and cooling. Multiple means may be combined.

Among the above means, chemical stimulation with a cell detachment solution is preferred from the viewpoint of the quality of the detached cells. Among these, chemical stimulation with a cell detachment solution substantially free of a proteolytic enzyme is particularly preferred.

Here, the proteolytic enzyme is an enzyme that decomposes a portion of the cell to facilitate the detachment of the cell from the substrate. Examples of the proteolytic enzyme include trypsin, accutase, collagenase, natural protease, chymotrypsin, elastase, papain, pronase, and recombinants thereof. The phrase "substantially free of a proteolytic enzyme" indicates that the amount of proteolytic enzyme based on the total mass of the cell detachment solution is 0.0005% or less by mass.

With regard to the state in which the cells adhere to the culture container, the cells are observed while shaking the culture container, and whether the cells adhere to the culture container can be determined based on the fact that the cells do not move. In addition, in the step of reducing the adhesion strength of cells, some cells having weak adhesion strength, such as cells before and after cell division, may be detached.

(Cell Detachment Solution)

The cell detachment solution is a solution used to reduce the adhesion strength of cells.

The pH of the cell detachment solution is preferably in a neutral range. Here, the neutral range refers to a pH of 6 or more and a pH of 8 or less. This is because the neutral range is suitable for cell culture and can stably maintain a high cell survival rate. The pH can be appropriately adjusted with, for example, hydrochloric acid or sodium hydroxide. To stably maintain the pH, various buffer solutions are suitably used.

The viscosity of the cell detachment solution is preferably 1.80 mPa·s or less. The viscosity of the cell detachment solution can be appropriately adjusted by adding polymer or sugar.

As the cell detachment solution, a solution containing a metal ion chelating agent (hereinafter, sometimes referred to as a "chelating agent") is particularly preferred. This is because the use of the cell detachment solution containing a chelating agent can effectively reduce the adhesion strength of cells.

The chelating agent is not particularly limited. Examples of the chelating agent include at least one selected from the group consisting of ethylenediaminetetraacetic acid (hereinafter, sometimes referred to as "EDTA"), ethylenediamine, ethylenediaminetetra(methylenephosphonic acid), glycoletherdiaminetetraacetic acid, nitrilotriacetic acid, diethylenetriaminepentaacetic acid, iminodiacetic acid, dihydroxyethylglycine, dicarboxymethylglutamic acid, ethylenediaminedisuccinic acid, etidronic acid, citric acid, gluconic acid, and phosphonobutanetriacetic acid. Among these, chelating agents that form chelates with divalent cations are preferred. Chelating agents that form chelates with $Ca^{2+}$ and $Mg^{2+}$ are particularly preferred. Ethylenediaminetetraacetic acid is most preferred.

When ethylenediaminetetraacetic acid is used as a chelating agent, the pH of the cell detachment solution is preferably 7.0 or more and 8.0 or less. This is because the chelating ability of ethylenediaminetetraacetic acid can be enhanced and the efficiency of reducing the adhesion strength of the cells can be further increased by setting the pH to a relatively high value in the neutral range where a high cell survival rate can be maintained. The chelating agents may be used alone or in combination of two or more.

The chelating agent content is preferably 0.01 mM or more and 5.0 mM or less. Within this range, a chelating effect can be reliably provided, and a deterioration in the proliferation ability of cells due to the presence of an excess of the chelating agent can be reduced.

The cell detachment solution may contain a hydrophilic polymer containing a polyalkylene glycol structure. Examples of the hydrophilic polymer containing a polyalkylene glycol structure include polyethylene glycols. The hydrophilic polymer preferably has a peak molecular weight Mp of 800 or more and 50,000 or less, more preferably 1,200 or more and 20,000 or less, measured by gel permeation chromatography. This is because the influence of the polymer on the cells is small and the thickening effect of the polymer can be reduced.

(Buffer Solution)

The buffer solution used for the cell detachment solution can be used without any limitation as long as the neutral range can be maintained. Examples of the buffer solution include a Tris buffer solution, such as a Tris-HCl buffer solution, a phosphate buffer solution, a HEPES buffer solution, a citrate-phosphate buffer solution, a glycylglycine-sodium hydroxide buffer solution, a Britton-Robinson buffer solution, and a GTA buffer solution. Among these, a phosphate buffer solution close to the environment in a living body is preferred. A phosphate-buffered saline (PBS) prepared so as to be isotonic with an intracellular fluid is more preferably used as the phosphate buffer solution.

In the adhesion strength reduction step according to an embodiment of the present invention, when chemical stimulation with a cell detachment solution is used, in other words, when an incubation step of bringing cells into contact with the cell detachment solution and incubating the cells is provided, the incubation step preferably includes the following steps.

[1-1] A step of bringing cells into contact with a cell detachment solution while the cells remain adhered to a substrate.
[1-2] A step of performing incubation.

(Step [1-1])

Step [1-1] is a step of replacing the growth medium used for cell culture with a cell detachment solution. The cells may be washed with a buffer solution before or after step [1-1]. For example, when cultured cells are detached from a cell culture container, the effect of the cell detachment solution can be increased by first removing the growth medium and washing the cells with the buffer solution before adding the cell detachment solution while the cells remain adhered to the cell culture container. This is because the growth medium typically contains a factor, such as serum, which inhibits a reduction in cell adhesion.

(Step [1-2])

Incubation in the step [1-2] refers to performing a step including at least one of a step of adjusting the temperature, a step of adjusting the humidity, and a step of adjusting the air composition. The step of adjusting the temperature is, for example, a step of controlling a heater in such a manner that the temperature around the cell culture container is about 37°C. The step of adjusting the humidity is, for example, a step of adjusting the humidity to 90% or more by installing a humidifying vat in such a manner that the medium does not dry. The step of adjusting the air composition is, for example, a step of supplying $CO_2$ in such a manner that the $CO_2$ partial pressure is about 5% in order to prevent oxidation of the culture medium.

The incubation time is, but not particularly limited to, for example, in the range of 30 seconds to 20 minutes. The incubation temperature is not particularly limited, but is preferably 10°C to 40°C from the viewpoint of maintaining the cell survival rate. In particular, 30°C to 40°C is the optimum temperature for cell growth and is preferred.

(Measurement Step)

The measurement step in the cell detachment method according to an embodiment of the present invention is a step of acquiring the cell information of the cells adhering to the cell culture container. The measurement step (acquisition of cell information) may be performed at a time point before the step of reducing the adhesion strength of the cells is performed. The acquisition of the cell information may be performed at at least two time points. The acquisition of the cell information may be performed multiple times at regular intervals. Of the two time points at which the cell information is acquired, a later time point is defined as a time point t1. The time point t2 at which a detachment step described below is to be started is a time point after the time point t1.

In the measurement step, time-series data relating to the cells may be acquired. The time-series data indicates cell information measured at at least two time points and a time point at which the cell information is measured.

The number of time instances at which the cell information is acquired is preferably 2 or more and 10,000 or less, more preferably 3 or more and 1,000 or less, and still more preferably 5 or more and 300 or less. When the number of time instances at which the cell information is acquired is two or more, it is possible to accurately determine the start time point of the detachment step. In addition to the cell information at the time instances at which the cell information is actually acquired, for example, cell information acquired in advance under the same conditions may be used as the cell information at the start time point of the adhesion strength reduction step. When the number of time instances at which the cell information is acquired is 10,000 or less, the processing time to determine the start time point of the detachment step can be reduced.

Examples of the cell information measured in the measurement step include the area of the cells, the area of a halo region in a phase-contrast microscope image (also referred to as a "phase-contrast image"), a brightness value in the phase-contrast image, and a brightness value when a cell culture surface of the cell culture container is observed under oblique-incidence illumination.

As an example of the area of the cells, for example, a phase-contrast image of adhering cells is acquired, and an area occupied by a single cell is measured using image processing, thereby making it possible to digitize the area of the cell.

It is known that an adhering cell appears to have a large area in a phase-contrast image, whereas a cell having reduced adhesion strength appears to have a small area in a phase-contrast image because the cell has a shape close to a spherical shape due to surface tension. Using this phenomenon, the area of the cell on the phase-contrast image is digitized, and the change thereof is observed.

Another type of cell information is a brightness value or the area of the halo region in the phase-contrast image.

In the phase-contrast image of the cells in the adherent state, only a region having a small phase difference as a whole (a state where the cells are flat) is detected. In contrast, it is known that a halo region is observed in which the adhesion of the cells is weakened, the phase difference is increased in the periphery where the cells are lifted from the culture surface, and the image brightness value is higher than other regions. That is, when the adhesion of the cells is weakened, the area of the halo region increases.

An example of another type of cell information is a brightness value when the cell culture surface of the cell culture container is observed under oblique-incidence illumination. In the case of a cell having reduced adhesion strength, the pseudopodium shrinks and the microscopic surface adhering to the cell culture container is reduced, so that light is easily scattered at the interface between the cell culture container and the cell. Thus, when observation is performed under oblique-incidence illumination in which the proportion of scattered light increases, if the adhesion strength of the cell decreases, the brightness value of the cell culture surface of the cell culture container increases.

In the embodiment, various parameters can be used as the cell information. The cell information may be, for example, at least one selected from the group consisting of the area of each cell, the size of each cell, the roundness of each cell, the light transmittance of each cell, the occupied area of a cell population, the brightness value of a cell image, the area of a region where no cells are present, the area of a halo region, the brightness value of a halo-based image, and the brightness value of an image obtained by observing a cell culture surface of a cell culture container under oblique-incidence illumination.

In the embodiment, the cell information can be measured in various ways. For example, it is possible to measure various types of the cell information by acquiring a phase-contrast image of a cell using a phase-contrast microscope and analyzing the image. In addition, it is possible to measure the brightness value of the culture surface by illumination means to illuminating the cell culture surface of the cell culture container at an oblique incidence and observation means arranged in a direction perpendicular to the culture surface. The illumination means and the observation means may be arranged at positions opposite each other across the cell culture container or may be arranged in the same direction as long as they are arranged so as to easily receive the scattered light on the culture surface.

The cell information is preferably brightness information relating to cells, more preferably brightness information of a cell image. The brightness information is preferably brightness information of a culture surface observed under oblique-incidence illumination. The oblique-incidence illumination is preferably ring illumination from the viewpoint of reducing unevenness in brightness. The brightness information is preferably the average brightness value of the culture surface or the area of the culture surface having a brightness value exceeding a threshold value.

The observation means to measure the cell information preferably has a field of view of 1 cm$^2$ or more. The use of a field of view of 1 cm$^2$ or more enables a reduction in the adhesion strength of the cells to be evaluated on a macroscopic scale without being affected by a micro-deviation in the culture state of the cells.

(Example of Measuring Area of Cell as Cell Information)

Immediately after the growth medium was replaced with a cell detachment solution, a phase-contrast image of the cell was acquired with time. A specific cell was marked, and the area of this cell was quantified using image processing software.

The cell area thus determined decreases with time as the adhesion of the cell to the cell culture container weakens. For example, the cell area, which was 237 $\mu$m$^2$, was reduced to 113 $\mu$m$^2$ after 10 minutes of incubation.

(Example of Measuring Rate of Change in Cell Area as Cell Information)

Immediately after the growth medium was replaced with a cell detachment solution, a phase-contrast image of the cell was acquired with time. A specific cell was marked, and the area of this cell was quantified using image processing software. The area of the cells immediately after contact with the cell detachment solution (incubation time: zero) is defined as A1. The area of the cells at an incubation time point t in the cell detachment solution is defined as A2. The rate of change in cell area was calculated by the following Expression (1).

$$(A1 - A2)/A1 \cdots (1)$$

The rate of change in cell area thus determined increases with time as the adhesion of the cell to the substrate weakens. For example, the rate of change in cell area was 0.15 after 1 minute of incubation, but increased to 0.5 after 5 minutes of incubation.

(Example of Measuring Area Fraction of Halo Region as Cell Information)

Immediately after the growth medium was replaced with a cell detachment solution, a phase-contrast image of the cell was acquired with time. The area fraction of a halo region was measured by performing binarization, setting of the threshold value, and area measurement of the phase-contrast image of these cells using image processing software. Here, a certain brightness value on the image was set as a threshold value. A region having a brightness value higher than or equal to the threshold value was defined as a halo region. The area of the halo region in the area of the entire image was determined.

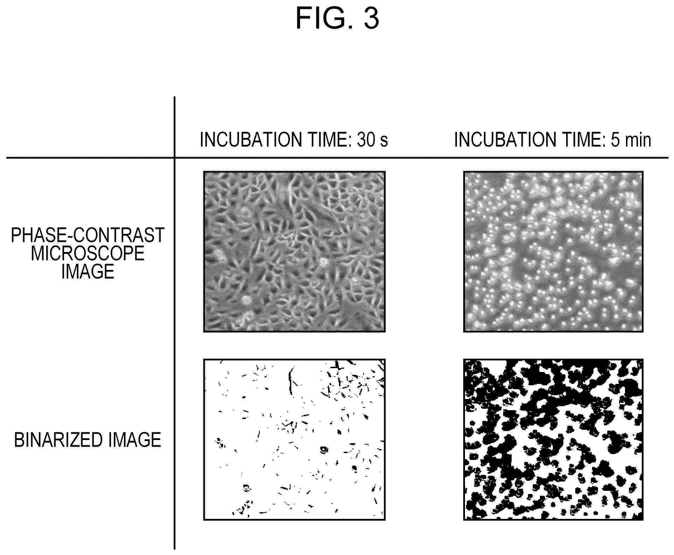

The area fraction of the halo region thus obtained increases with time as the adhesion of the cells to the substrate weakens. Fig. 3 illustrates an example of the acquired phase-contrast microscope image and the binarized image. In the binarized image, the halo region is represented in black. For example, the area fraction of the halo region in the phase-contrast image at the incubation time of 30 seconds in the cell detachment solution was 2.8%, and the area fraction of the halo region in the phase-contrast image at the incubation time of 5 minutes in the cell detachment solution increased to 49.9%.

(Example of Measuring Brightness Value of Phase-Contrast Image as Cell Information)

Immediately after the growth medium was replaced with the cell detachment solution, the phase-contrast image of the cells was acquired with time. The brightness value of these cells in the phase-contrast image was measured using image processing software.

The brightness value of the phase-contrast image obtained in this way increases with time because the halo region increases as the adhesion of the cells to the substrate weakens. For example, the brightness value of the phase-contrast image at an incubation time of 30 seconds in the cell detachment solution was 7.2, and the brightness value of the phase-contrast image at an incubation time of 5 minutes in the cell detachment solution increased to 127.2.

(Example of Measuring Brightness Value of Image Observed Under Oblique-Incidence Illumination as Cell Information)

Immediately after the growth medium was replaced with a cell detachment solution, an image of the cell culture surface observed under oblique-incidence illumination was acquired with time. The brightness value of the entire cell culture surface of the cell culture container was measured using image processing software.

The brightness value of the cell culture surface observed under the oblique-incidence illumination thus obtained increases with time because the intensity of scattered light increases as the adhesion of the cells to the cell culture container weakens. For example, when the incubation time in the cell detachment solution was 30 seconds, the average value of the brightness of the entire culture surface of the cell culture container was 96.2. When the incubation time in the cell detachment solution was 5 minutes, the average value of the brightness of the entire culture surface of the cell culture container increased to 108.0.

(Determination Step)

The determination step in the cell detachment method according to an embodiment of the present invention is a step of determining, based on cell information measured at at least two time points, a time point at which the detachment operation is to be started, the time point being after a time point at which the cell information has been measured. The cell detachment method according to an embodiment of the present invention may also include a prediction step of predicting, based on time-series data relating to the cells, a time point at which the detachment step is to be started. The time point used here may be an absolute time point, such as 13:30. The start time point determined may be a relative time point, such as 30 seconds after any one of the start time point of the incubation step, the start time point of the acquisition of the cell information, and the time instance when the start time is determined.

The interval at which cell information is measured is preferably 1 second or more and 1 minute or less. That is, the interval between the at least two time points at which the cell information is acquired is preferably 1 second or more and 1 minute or less. The measurement of the cell information at an interval within this range can accurately determine the start time point t2 of the detachment operation. The measurement intervals may be equal or unequal.

The time point t1 at which the cell information is measured is preferably 1 hour or less from the start time point t0 of the incubation (start time point of the adhesion strength reduction step), particularly preferably 15 minutes or less from t0. The time point t1 at which the cell information is measured is preferably 10 seconds or more from the start time point t0 of the incubation (start time point of the adhesion strength reduction step), particularly preferably 30 seconds or more from t0. When the time point t1 is within this range, a decrease in cell survival rate can be reduced.

t0, t1, and t2 preferably satisfy the relationship (t2 - t0) /(t1 - t0) ≥ 1.1. t0 is the start time point of the adhesion strength reduction step. When this relationship between t1 and t2 is satisfied, the preliminary operation time can be sufficiently ensured until the start time point of the detachment.

The determination step may include a step of determining an end time point t3 of the detachment operation. A method for determining t3 is not particularly limited. An example thereof is a method in which t3 is determined by a constant multiple of a value obtained by curve fitting based on time-series data of the cell information.

(Curve Fitting)

In the determination step, preferably, a fitted curve is obtained based on the cell information and the time point at which the cell information is acquired, and the time point t2 is determined based on the fitted curve. In a determination method, a fitted curve is preferably obtained using curve fitting. A function used for curve fitting and indicating the fitted curve is not particularly limited. An appropriate function is used in accordance with the cell type used, the cell information, and the means and conditions to reduce the adhesion strength of the cells. Examples of the function include functions represented by the following Expressions (2-1) to (2-9).

[Math. 1]

$$f(t) = C_0 + C_1 \left( 1 - \exp\left( - \left( \frac{t}{\tau} \right)^{\beta} \right) \right) \cdot \cdot \cdot (2-1)$$

[Math. 2]

$$f(t) = C_0 - C_1 \left( 1 - \exp\left( - \left( \frac{t}{\tau} \right)^{\beta} \right) \right) \cdot \cdot \cdot (2-2)$$

[Math. 3]

$$f(t) = C_0 + C_1 \exp\left( - \left( \frac{t}{\tau_1} \right)^{\beta_1} \right) + C_2 \exp\left( - \left( \frac{t}{\tau_2} \right)^{\beta_2} \right) \cdot \cdot \cdot (2-3)$$

[Math. 4]

$$f(t) = C_0 + C_1 \log\left( t + \exp\left( -\frac{C_2}{C_1} \right) \right) + C_2 \cdot \cdot \cdot (2-4)$$

[Math. 5]

$$f(t) = C_0 + \frac{C_1}{1 + \exp\left( -\frac{t-\tau}{C_2} \right)} \cdot \cdot \cdot (2-5)$$

[Math. 6]

$$f(t) = C_0 + \frac{C_2 t}{t + C_1} \cdot \cdot \cdot (2-6)$$

[Math. 7]

$$f(t) = C_0 + \frac{C_2 t}{\sqrt{t^2 + C_1}} \cdot \cdot \cdot (2-7)$$

[Math. 8]

$$f(t) = C_0 \alpha^{\beta^t} \cdot \cdot \cdot (2-8)$$

$$f(t) = C_0 + C_1 t + C_2 t^2 + C_3 t^3 + C_4 t^4 \cdot \cdot \cdot (2-9)$$

Here, t is a time point at which the cell information is measured when the time point at which the step of reducing the adhesion strength of the cells is started is set to **0,** and f(t) is the cell information measured at the time point t.

The values of parameters other than t in Expressions (2-1) to (2-9) may be determined in advance or may be determined based on the time-series data of the cell information, and at least one fitting parameter determined by the curve fitting of the time-series data of the cell information is provided. As an example, in Expression (2-1), all of $C_0$, $C_1$, $\tau$, and $\beta$ may be used as fitting parameters. Alternatively, $C_0$ may be cell information at the time point **0,** $\beta$ may be determined in advance, and $C_1$ and $\tau$ may be used as fitting parameters.

As the cell information used for curve fitting, cell information measured up to the time point t1 is used. Cell information acquired by thinning out appropriate time points from the time points at which the cell information has been measured may be used. Cell information at all time points at which measurement has been performed may be used. Before performing curve fitting, smoothing processing, such as filtering processing, may be performed on the time-series data of the cell information.

A method for determining the value of a fitting parameter by curve fitting is not particularly limited, and examples thereof include a least-squares method and a least absolute deviation method.

(Determination of Start Time Point t2)

After the time-series data of the cell information is fitted by the curve fitting, the time point t2 at which the detachment operation is to be started is determined. The time point t2 is later than the time point t1 at which the measurement step is performed. A method for determining the time point t2 is not particularly limited. As an example, a method will be described in which the average value of the brightness of the image observed under oblique-incidence illumination is used as the cell information, and the function used for curve fitting is the function represented by Expression (2-1) where $\beta = 1$.

(Determination Example 1 of Start Time Point t2)

A brightness threshold value Th1 at which the detachment operation is started is provided in advance in accordance with the type of cell, the culture conditions, and the detachment operation. The average value of the brightness is measured until the time point t1. The time point when the curve of the function obtained by curve fitting using Expression (2-1) reaches Th1 is calculated. This time point is defined as the start time point t2.

(Determination Example 2 of Start Time Point t2)

The average value of brightness is measured until the time point t1. The values of the fitting parameters $C_0$, $C_1$, and $\tau$ in Expression (2-1) are calculated by curve fitting. The time point $A_\tau$, which is A times of $\tau$, is defined as the start time point t2. Here, A is provided in advance in accordance with the type of cell, the culture conditions, and the detachment operation. A is preferably 0.3 or more and 2 or less, particularly preferably 0.5 or more and 1 or less.

(Determination Example 3 of Start Time Point t2)

The average value of brightness is measured until the time point t1. The time point when the curve of the function obtained by curve fitting using Expression (2-1) reaches $C_0 + C_1 \times B$ is calculated. This time point is defined as the start time point t2. Here, B is provided in advance in accordance with the type of cell, the culture conditions, and the detachment operation. B is a positive value of less than 1. B is preferably 0.3 or more and 0.9 or less, particularly preferably 0.4 or more and 0.7 or less.

(Detachment Operation)

The detachment operation to be started at the time point t2 determined in the determination step includes vibration induced by ultrasound, vibration by tapping, convection to a culture solution or the cell detachment solution, and the like. Multiple means may be combined. Examples of a method for generating convection to the culture solution or the cell detachment solution include pipetting and using a pump or a mixing impeller. From the viewpoint of the quality of the cells after detachment, vibration induced by ultrasound is preferably used for the detachment operation.

(Vibration Induced by Ultrasound)

An example of the vibration induced by ultrasound is vibration having a frequency of about 10 kHz to 1 MHz, as described in International Publication No. 2016-047368. A vibration generating means can be used without particular limitations as long as it can subject the cells to vibration induced by ultrasound. For example, an ultrasonic transducer made of lead zirconate titanate (PZT) or the like can be used as described in Japanese Patent Laid-Open No. 2008-92857.
As described in Japanese Patent Laid-Open No. 2006-314204, a vibrator can be brought into direct contact with the outer surface of a sealed culture container filled with a medium to subject the culture container to vibration. In addition, as described in International Publication No. 2016-047368, instead of bringing ultrasound-emitting means into direct contact with a container, an ultrasound-transmitting substance can be interposed between the ultrasound-emitting means and the region to be treated to make ultrasound incident on the cells to be detached.
The time to subject the cells to vibration induced by ultrasound is, but not particularly limited to, for example, 1 second to 1 hour, preferably 5 seconds to 30 minutes, and more preferably 10 seconds to 15 minutes, from the viewpoint of effectively detaching the cells and increasing the cell survival rate.
The environmental temperature at which the cells are subjected to vibration induced by ultrasound is preferably, but not particularly limited to, 20°C to 40°C, particularly preferably 30.0°C to 37.5°C, from the viewpoint of maintaining the cell survival rate. This is because the temperature at the time of cell detachment is close to the temperature at the time of culture, making it possible to reduce the influence of a change in temperature on the cells and maintain a high survival rate.

(Treatment After Cell Detachment)

The cells detached by the detachment operation may be seeded in a culture container such as a new substrate, dish, or flask, or may be subjected to a step, such as labeling of the cells. The cells may also be used as a sample for measurement of the number of cells or the cell survival rate. Furthermore, when the resulting cells associate with each other to form a cell mass, a cell dissociation treatment may be performed as appropriate.

(Configuration of Information Processing System)

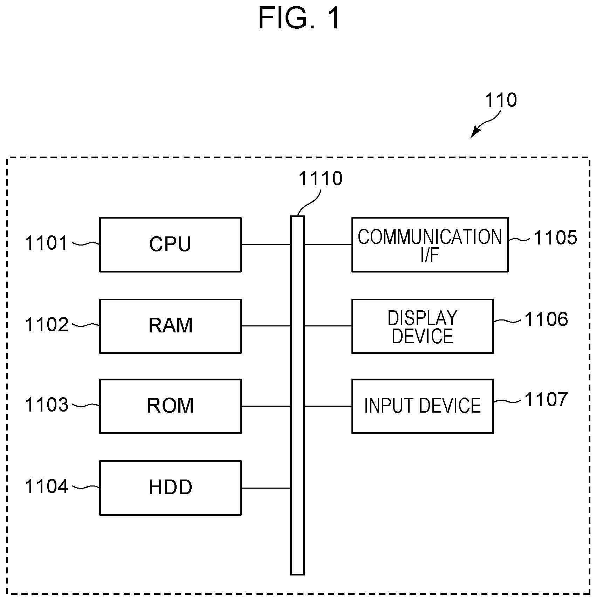

Fig. 1 is a block diagram illustrating a hardware configuration example of an information processing system 110 capable of executing a program according to an embodiment of the present invention.
The information processing system 110 has a function of a computer. For example, the information processing system 110 may be configured to be integrated with a desktop personal computer (PC), a laptop PC, a tablet PC, a smartphone, or the like.
The information processing system 110 implements a function as a computer that performs calculation and storage. The information processing system 110 includes a central processing unit (CPU) 1101, random access memory (RAM) 1102, read-only memory (ROM) 1103, and a hard disk drive (HDD) 1104. In addition, the information processing system 110 includes a communication interface (I/F) 1105, a display device 1106, and an input device 1107. The CPU 1101, the RAM 1102, the ROM 1103, the HDD 1104, the communication I/F 1105, the display device 1106, and the input device 1107 are connected to each other through a bus 1110. The display device 1106 and the input device 1107 may be connected to the bus 1110 through a driving device, not illustrated in the drawing, to drive these devices.
In Fig. 1, the devices constituting the information processing system 110 are illustrated as an integrated apparatus, but some of these functions may be implemented using external devices. For example, the display device 1106 and the input device 1107 may be external devices different from those constituting the functions of the computer including the CPU 1101 and the like.
The CPU 1101 performs predetermined operations according to a program stored in the RAM 1102, the HDD 1104, and the like, and also has a function of controlling each device of the information processing system 110. The RAM 1102 includes a volatile storage medium and provides a temporary memory region necessary for the operation of the CPU 1101. The ROM 1103 includes a nonvolatile storage medium and stores necessary information, such as a program used for the operation of the information processing system 110. The HDD 1104 includes a nonvolatile storage medium and is a storage device for storing information on the number and positions of individual independent separate compartments, fluorescence intensity, and the like.

The communication I/F 1105 is a communication interface in accordance with standards, such as Wi-Fi (registered trademark) and 4G, and is a module for communicating with other devices. The display device 1106 is a liquid crystal display, an organic light-emitting diode (OLED) display, or the like, and is used for displaying moving images, still images, characters, and the like. The input device 1107 is a button, a touch panel, a keyboard, a pointing device, or the like, and is used by a user to operate the information processing system 110. The display device 1106 and the input device 1107 may be integrally formed as a touch panel.

The hardware configuration illustrated in Fig. 1 is an example, and devices other than these devices may be added, or some of the devices may not be provided. Some of the devices may be replaced with other devices having the same function. Furthermore, some of the functions may be provided by other devices via a network, and the functions included in the embodiment may be distributed and implemented by multiple devices. For example, the HDD 1104 may be replaced by a solid-state drive (SSD) using a semiconductor device, such as flash memory, or may be replaced with cloud storage.

(Cell Detachment System)

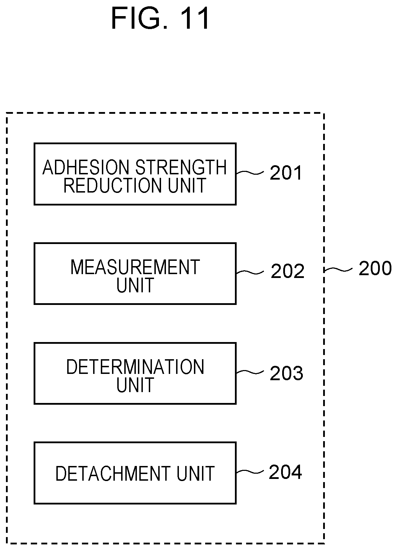

A cell detachment system according to an embodiment of the present invention includes an adhesion strength reduction unit 201 to reduce the adhesion strength of a cell, a measurement unit 202 to measure the cell at at least two time points and acquire cell information, a detachment unit 204 to detach the cell from a culture surface of a cell culture container, and a determination unit 203 to determine, based on the cell information, a time point at which a step of detaching the cell is to be started. Fig. 11 is a schematic view of a cell detachment system. A cell detachment system 200 may be a cell detachment apparatus described below. In addition, the cell detachment system 200 may include multiple devices, and the functions of the cell detachment system 200 may be implemented by these devices.

(Cell Detachment Apparatus)

An example of a cell detachment apparatus according to an embodiment of the present invention is a cell detachment apparatus to detach cells cultured on a culture surface of a cell culture container. The cell detachment apparatus includes a medium exchange unit to bring cells into contact with a cell detachment solution while the cells remain adhered to the cell culture container, a cell information acquisition unit to acquire and measure the cell information of the cells, an ultrasonic irradiation determination unit to determine the timing of ultrasonic irradiation, and an ultrasonic irradiation unit to subject the cells to vibration induced by ultrasound.

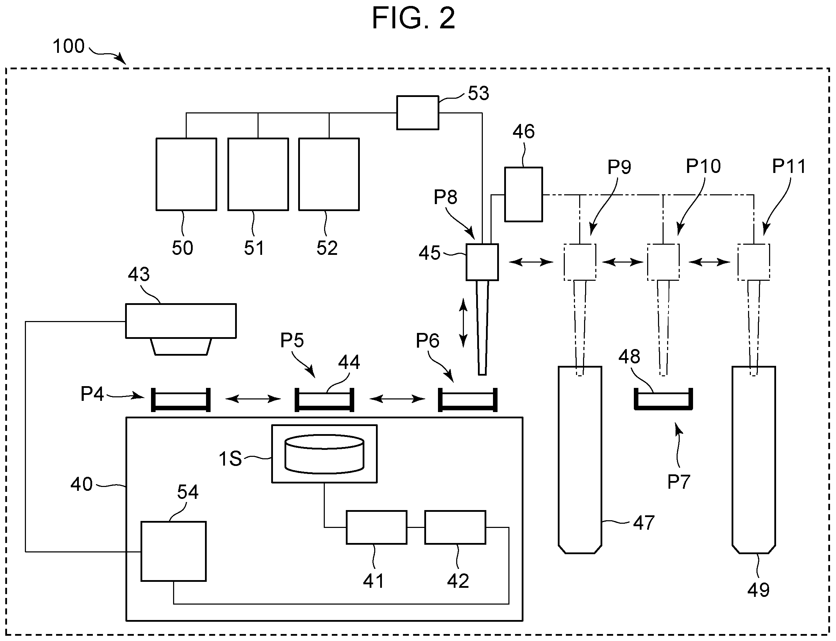

An example of the configuration of a cell detachment apparatus 100 according to the embodiment of the present invention will be described using the conceptual diagram of Fig. 2. The components in Fig. 2 are 100: the cell detachment apparatus, 1S: an ultrasonic irradiation unit, 40: a culture container movement controller, 41: an amplifier, 42: a function generator, 43: a cell information acquisition unit (which may include a cell information analysis unit), 44: a cell culture container, 45: a pipetting unit, 46: a pipettor movement controller, 47: a waste liquid recovery unit, 48: a detached cell recovery unit (cell culture container), 49: a detached cell recovery unit (tube), 50: a washing medium, 51: a culture medium, 52: a cell detachment solution, 53: a liquid transport controller, and 54: an ultrasonic irradiation determination unit (which may include a cell information analysis unit). Positions are P4: the position of the culture container at the time of the acquisition of cell information, P5: the position of the culture container at the time of cell detachment (set in the ultrasonic irradiation unit), P6: the position of the culture container at the time of addition of the cell detachment solution, medium exchange, or cell recovery, P7: the position of the new culture container at the time of seeding the detached cells into a new culture container, P8: the position of the pipettor at the time of addition of the cell detachment solution, medium exchange, or cell recovery, P9: the position of the pipettor at the time of disposal of the medium or cell suspension, P10: the position of the pipettor at the time of seeding the cell suspension into a dish, and P11: the position of the pipettor at the time of recovering the cell suspension into a tube.

The culture container movement controller 40 is a unit to hold and move the culture container 44.

The culture container movement controller 40 may include a warmer to control the temperature and humidity of the cell detachment environment, and examples thereof include a heating unit and a humidifying unit which are capable of maintaining the temperature at 37°C. For moving the culture container 44, for example, a motorized XYZ stage is used. Alternatively, a robot arm to hold and move the culture container 44 may be used. The culture container movement controller 40 horizontally moves the culture container 44 between the position P4, the position P5, and the position P6. At the position P4, the cell information acquisition unit 43 can acquire the cell information, such as a phase-contrast image, of cells cultured in the culture container 44. At the position P5, the ultrasonic irradiation unit 1S irradiates the inside of the culture container 44 with ultrasonic waves. At the position P6, the pipetting unit 45 recovers the solution in the culture container 44 or adds various types of solutions (sometimes referred to as "culture media") to the culture container 44. The pipetting unit 45 may include a mechanism for automatically or manually discharging or aspirating the culture medium. The pipetting unit 45 is not limited in configuration as long as it can transport a liquid, and may be, for example, a tube pump. In addition, a plurality of pipetting units may be provided, and may be independently provided in accordance with the type or role of the medium.

Further explanation is given using an example of a process of cell detachment using ultrasonic waves.

Cells are cultured in the culture container 44, and the culture container 44 is placed at the position P6 when the timing at which the cells are to be detached is reached. The culture container 44 is appropriately placed at the position P4 by the culture container movement controller 40, and the state of the cells can be observed with the cell information acquisition unit 43. In the culture container 44 placed at the position P6, the cells adhere to the container. First, it is necessary to remove the medium and replace it with a cell detachment solution. For this reason, at the position P6, the medium in the culture container 44 is recovered with the pipetting unit 45. The recovered medium is discarded to the waste liquid recovery unit 47 at the position P9. Various media are stocked in a washing medium 50, a culture medium 51, and a cell detachment solution 52, and are transported to the pipetting unit 45 by the liquid transport controller 53. The pipetting unit can move vertically to access the culture container 44. A lid opening and closing system of the culture container 44 may be provided as appropriate. After removing the medium, the cells adhering to the container may be washed as appropriate. At the position P6, into the culture container 44 with the pipetting unit 45, the washing medium 50 is transported to the pipetting unit 45 by the liquid transport controller 53, and the washing medium 50 is added to the culture container 44 with the pipetting unit 45 at the position P6. After the washing medium 50 is removed in the same way as the medium removal method described above, the cell detachment solution 52 is transported to the pipetting unit 45 by the liquid transport controller 53, and at the position P6, the cell detachment solution 52 is added into the culture container 44 by the pipetting unit 45.

Next, the culture container movement controller 40 moves the culture container 44 to the position P4. Here, the cells are incubated in the cell detachment solution while the cell information acquisition unit 43 acquires the cell information. During the incubation, the cell information acquisition unit 43 acquires the cell information, the resulting cell information is analyzed, and the start time point of the detachment operation is determined by curve fitting based on the time-series data of the cell information. The determination is performed by the ultrasonic irradiation determination unit 54. Thereafter, the culture container movement controller 40 is instructed to move the culture container 44, and an instruction to activate the ultrasonic irradiation unit 1S, the function generator 42, and the amplifier 41 is transmitted.

Next, the culture container movement controller 40 moves the culture container 44 to the position P5. Here, the culture container 44 is placed at the ultrasonic irradiation unit 1S. An example configuration is one in which an ultrasonic detachment unit is combined with the function generator 42 and the amplifier 41, which input a freely-selected frequency, voltage, and so forth to the ultrasonic detachment unit. At the determined start time point, the culture container 44 is irradiated with ultrasonic waves from the ultrasonic irradiation unit 1S, and the cells adhering to the culture container 44 are detached. The culture container 44 may be appropriately moved to the position P4, and the cell information acquisition unit 43 may be used to monitor the detached state of the cells.

After the detachment of the cells is completed, the culture container movement controller 40 moves the culture container 44 to the position P6. At the position P6, the cell detachment solution containing the detached cells in the culture container 44 is recovered by the pipetting unit 45. At the position P10, the cell detachment solution containing the recovery detached cells is seeded in the detached cell recovery unit 48, for example, in a new cell culture container placed at the position P7. Alternatively, at the position P11, the cells are recovered in the tube 49, which is the detached cell recovery unit. These recovered cells are subsequently used for cell culture and assays, such as cell measurement and cell labeling.

The cell detachment apparatus according to an embodiment of the present invention may further include a controller to control the cell detachment process. The controller is means to control the operation of each unit in the cell detachment apparatus 100. For example, the controller may include a computer including an arithmetic processing unit, such as a CPU, memory, such as RAM, and a storage unit, such as a hard disk drive.

The controller may also include an operation unit. The operation unit may include a display unit, an input unit, and so forth. The display unit can display image data output from the controller and various pieces of information relating to the operation of the cell detachment apparatus 100. For example, a liquid crystal display is used as the display unit. The input unit receives various instruction inputs from a user. For example, a keyboard or a mouse is used as the input unit. Both the function of the display unit and the function of the input unit may be in a single unit, such as a touch panel display.

(EXAMPLES)

The present invention will be described below in more detail with reference to examples and comparative examples, but the present invention is not limited thereto.

[Example 1]

(Culture of Cell on Substrate)

Chinese hamster ovary (CHO) cells were seeded in a ø35 polystyrene dish (manufactured by Corning Incorporated) at a density of 10,000 cells/cm$^2$, and cultured in an environment at 37°C and at a $CO_2$ concentration of 5%. A medium used was Ham's F12 (manufactured by Thermo Fisher Scientific Inc.) to which 10% of fetal bovine serum (manufactured by Sigma-Aldrich) and 1% of penicillin-streptomycin (10,000 U/ml, manufactured by Thermo Fisher Scientific Inc.) were added. The culture was performed for 48 hours. The state of the cells was observed with a phase-contrast microscope to confirm cell adhesion and proliferation. The proportion of the cell-occupied area of the dish was about 80%.

(Detachment of Cell)

The medium in the dish was removed, and then phosphate-buffered saline (PBS(-), manufactured by Thermo Fisher Scientific Inc.) was added to the dish as a cell detachment solution. An observation image of the cell culture surface of the dish was acquired under oblique-incidence illumination. The average brightness value of the entire cell culture surface of the dish was measured using image processing software. The area of the field of view of the image in this case was 50 cm$^2$. The measurement was started immediately after the cell detachment solution was added to the dish, and was performed while incubating the cells in an incubator at 37°C. The measurements were performed at intervals of 5 seconds for 2.5 minutes.

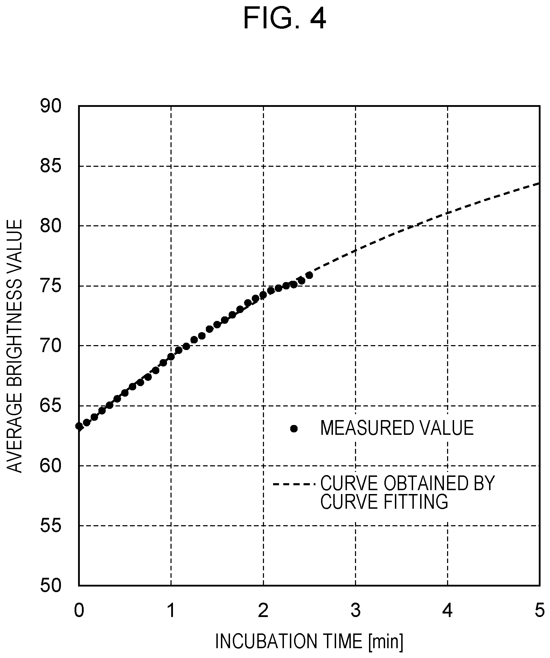

A temporal change in the average brightness value obtained from the measurement is illustrated in Fig. 4. Curve fitting was performed on this time-series data using the function represented by Expression (2-1). Here, the value of $\beta$ was set to 1, and the values of the fitting parameters $C_0$, $C_1$, and $\tau$ were determined by a least-squares method. The values of $C_0$, $C_1$, and $\tau$ obtained were 62.9, 30.4, and 4.4, respectively. A curve obtained by curve fitting is illustrated in Fig. 4. A time point at which the average brightness value reached 77.5 was set in advance as the start time point t2 of the detachment operation. The start time point t2 was calculated from the curve obtained by curve fitting and was found to be t2 = 2.9 minutes.

After measuring the cell information, the dish was placed at an ultrasonic detachment apparatus. The cells were detached by sweep vibration (frequency: 29.5 to 34.5 kHz, sweep period: 1 second, voltage: 100 V) for 3 minutes from the time point t2 at an environmental temperature of 37°C.

After the detached cells were recovered, the number of cells was measured using a hemocytometer. The survival rate was calculated using a viability test by trypan blue staining. The suspension of the detached cells was mixed with a 0.4% trypan blue solution at a ratio of 1:1 to stain the dead cells contained in the suspension. The suspension was injected into a hemocytometer (DHC-N01, manufactured by NanoEntek). The number of live cells and dead cells on the grid of the hemocytometer was counted by microscopic observation. In the cells detached by the vibration of the cell detachment apparatus, the ratio of the number of live cells to the total number of cells counted was calculated as a survival rate.

The dish subjected to the ultrasonic detachment was treated with a cell scraper to detach any cells that had not been detached by the ultrasonic waves. The suspension of the detached cells was injected into a hemocytometer (DHC-N01, manufactured by NanoEntek). The number of detached cells was measured by microscopic observation. The total number of cells detached by ultrasound and the number of cells subsequently detached with the cell scraper were combined to obtain the total number of detached cells. The ratio of the number of detached cells by ultrasound to the total number of detached cells was defined as the detachment efficiency and calculated. The effect of the present invention was determined to be achieved when both of the survival rate of the detached cells and the cell detachment efficiency were 95% or more.

[Example 2]

The cell survival rate and the cell detachment efficiency were evaluated in the same manner as in Example 1, except that the start time point t2 of the detachment operation was set to 0.7 times $\tau$. The start time point t2 of the detachment operation in this case was 3.1 minutes.

[Example 3]

The cell survival rate and the cell detachment efficiency were evaluated in the same manner as in Example 1, except that the start time point t2 of the detachment operation was set to a time point at which the average brightness value reached $C_0 + C_1 \times 0.5$. The start time point t2 of the detachment operation in this case was 3.0 minutes.

[Example 4]

Cells were cultured in the same manner as in Example 1.

The medium in the dish was removed, and then phosphate-buffered saline (PBS(-), manufactured by Thermo Fisher Scientific Inc.) was added to the dish as a cell detachment solution. The dish was placed on the stage of a phase-contrast microscope to acquire a phase-contrast image. The average brightness value of the phase-contrast image was measured using image processing software. The area of the field of view of the image in this case was 6 mm$^2$. The measurement was started immediately after the cell detachment solution was added to the dish, and was performed while incubating the cells in an incubator at 37°C. The measurements were performed at intervals of 15 seconds for 2.5 minutes.

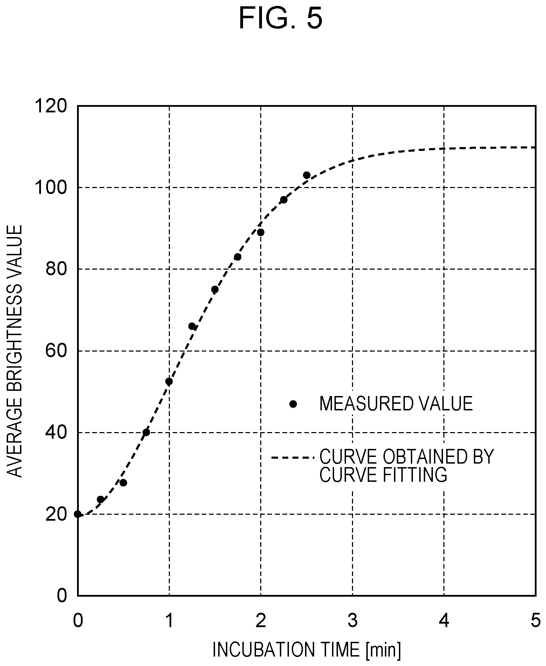

A temporal change in the average brightness value obtained from the measurement is illustrated in Fig. 5. Curve fitting was performed on this time-series data using the function represented by Expression (2-1). The values of the fitting parameters $C_0$, $C_1$, $\tau$, and $\beta$ were determined by a least-squares method. The resulting values of $C_0$, $C_1$, $\tau$, and $\beta$ were 19.5, 90.4, 1.6, and 1.8, respectively. A curve obtained by curve fitting is illustrated in Fig. 5. A time point at which the average brightness value reached 105 was set in advance as the start time point t2 of the detachment operation. The start time point t2 was calculated from the curve obtained by curve fitting and was found to be t2 = 2.8 minutes.

After measuring the cell information, the dish was placed in an ultrasonic detachment apparatus and subjected to sweep vibration (frequency: 29.5 to 34.5 kHz, sweep period: 1 second, voltage: 100 V) for 3 minutes from the time point t2 at an environmental temperature of 37°C. Thereafter, the cell survival rate and the cell detachment efficiency were evaluated in the same manner as in Example 1.

[Example 5]

Cells were cultured in the same manner as in Example 1.

The medium in the dish was removed, and then phosphate-buffered saline (PBS(-), manufactured by Thermo Fisher Scientific Inc.) was added to the dish as a cell detachment solution. The dish was placed on the stage of a phase-contrast microscope to acquire a phase-contrast image. The cell area of multiple cells was measured from the phase-contrast image using image processing software. The area of the field of view of the image in this case was 6 mm$^2$. The measurement was started immediately after the cell detachment solution was added to the dish, and was performed while incubating the cells in an incubator at 37°C. The measurements were performed at intervals of 15 seconds for 2 minutes. The measured cell areas of the multiple cells were averaged. The area of the cell immediately after the multiple cells came into contact with the cell detachment solution (incubation time: zero) was defined as A1.

Thereafter, the area of the cells at the incubation time t was defined as A2. The rate of change X in cell area was calculated by Expression (3) below.

$$X = (A1 - A2)/A1 \cdots (3)$$

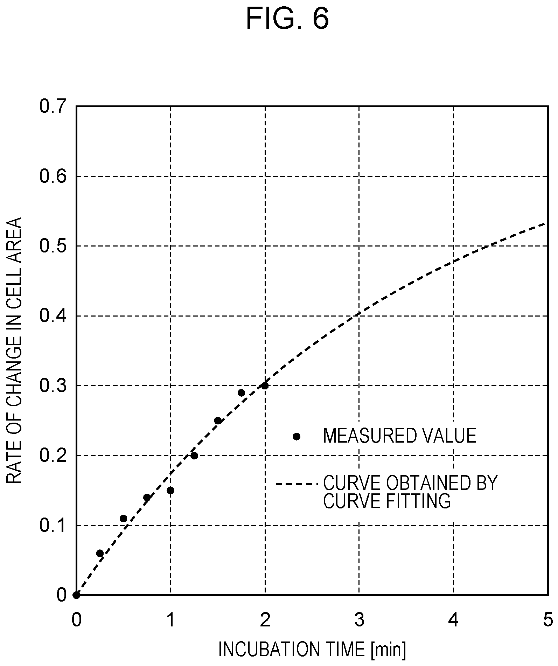

The rate of change in cell area with time obtained from the measurement is illustrated in Fig. 6. Curve fitting was performed on this time-series data using the function represented by Expression (2-1). Here, the value of $C_0$ was 0, the value of $\beta$ was 1, and the values of the fitting parameters $C_1$ and $\tau$ were determined by a least-squares method. The resulting values of $C_1$ and $\tau$ were 0.70 and 3.5, respectively. A curve obtained by curve fitting is illustrated in Fig. 6. A time at which the rate of change in cell area reached 0.4 was set in advance as the start time point t2 of the detachment operation. The start time point t2 was calculated from the curve obtained by curve fitting and was found to be t2 = 2.9 minutes.

After measuring the cell information, the dish was placed in an ultrasonic detachment apparatus and subjected to sweep vibration (frequency: 29.5 to 34.5 kHz, sweep period: 1 second, voltage: 100 V) for 3 minutes from the time point t2 at an environmental temperature of 37°C. Thereafter, the cell survival rate and the cell detachment efficiency were evaluated in the same manner as in Example 1.

[Comparative Examples 1 to 5]

The cell survival rates and the cell detachment efficiencies were evaluated in the same manner as in Example 1, except that the measurement of the cell information was not performed, and each start time point t2 was changed as given in Table 1.

Table 1 presents the results of the survival rates of the detached cells and the detachment efficiencies of the cells in Examples 1 to 5 and Comparative Examples 1 to 5.

[Table 1]

Table 1

| | Start time point t2 [min] | Survival rate of detached cells [%] | Detachment efficiency of cells [%] |
|---|---|---|---|
| Example 1 | 2.9 | 97.2 | 96.0 |
| Example 2 | 3.1 | 96.9 | 95.9 |
| Example 3 | 3.0 | 97.1 | 95.4 |
| Example 4 | 2.8 | 96.7 | 95.1 |
| Example 5 | 2.9 | 97.6 | 95.5 |
| Comparative Example 1 | 0.5 | 76.7 | 35.4 |
| Comparative Example 2 | 1 | 80.1 | 68.5 |
| Comparative Example 3 | 2 | 94.6 | 85.4 |
| Comparative Example 4 | 8 | 94.9 | 96.5 |
| Comparative Example 5 | 15 | 93.2 | 97.4 |

[Example 6]

(Cell Culture)

C2C12 cells, a mouse skeletal myoblast cell line, were seeded in a ø35 polystyrene dish (manufactured by Corning Incorporated) at a density of 5,000 cells/cm$^2$ and cultured in an environment at 37°C and at a $CO_2$ concentration of 5%. The medium used was DMEM/F12 (manufactured by Thermo Fisher Scientific Inc.) to which 10% of Fetal Bovine Serum (manufactured by Sigma-Aldrich) and 1% of penicillin-streptomycin (10,000 U/ml, manufactured by Thermo Fisher Scientific Inc.) were added. The culture was performed for 48 hours. The state of the cells was observed with a phase-contrast microscope to confirm cell adhesion and proliferation. The proportion of the cell-occupied area of the dish was about 80%.

(Detachment of Cell)

The medium in the dish was removed, and then phosphate-buffered saline (PBS(-), manufactured by Thermo Fisher Scientific Inc.) was added to the dish as a cell detachment solution. An observation image of the cell culture surface of the dish was acquired under oblique-incidence illumination. The average brightness value of the entire cell culture surface of the dish was measured using image processing software. The area of the field of view of the image at this time was 50 cm$^2$. The measurement was started immediately after the cell detachment solution was added to the dish, and was performed while incubating the cells in an incubator at 37°C. The measurements were performed at intervals of 1 second for 5 minutes.

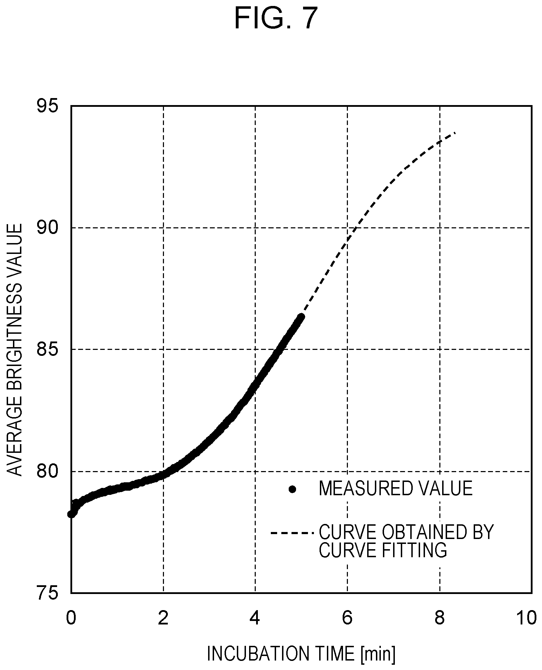

A temporal change in the average brightness value obtained from the measurement is illustrated in Fig. 7. Curve fitting was performed on this time-series data using the function represented by Expression (2-5). The values of the fitting parameters $C_0$, $C_1$, $C_2$, and $\tau$ were determined by a least-squares method. The resulting values of $C_0$, $C_1$, $C_2$, and $\tau$ were 78.4, 16.9, 1.34, and 5.15, respectively. A curve obtained by curve fitting is illustrated in Fig. 7. A time point at which the average brightness value reached 90.0 was set in advance as the start time point t2 of the detachment operation. The start time point t2 was calculated from the curve obtained by curve fitting and was found to be t2 = 6.2 minutes.

After measuring the cell information, the dish was placed at an ultrasonic detachment apparatus. The cells were detached by sweep vibration (frequency: 29.5 to 34.5 kHz, sweep period: 1 second, voltage: 100 V) for 3 minutes from the time point t2 at an environmental temperature of 37°C. Thereafter, the cell survival rate and the cell detachment efficiency were evaluated in the same manner as in Example 1.

[Comparative Examples 6 to 9]

The cell survival rates and the cell detachment efficiencies were evaluated in the same manner as in Example 6, except that the measurement of the cell information was not performed, and each start time point t2 was changed as given in Table 2.

Table 2 presents the results of the survival rates of the detached cells and the detachment efficiencies of the cells in Example 6 and Comparative Examples 6 to 9.

[Table 2]

Table 2

|  | Start time point t2 [min] | Survival rate of detached cells [%] | Detachment efficiency of cells [%] |
|---|---|---|---|
| Example 6 | 6.2 | 97.1 | 95.8 |
| Comparative Example 6 | 1 | 68.0 | 29.5 |
| Comparative Example 7 | 4 | 89.3 | 84.2 |
| Comparative Example 8 | 10 | 94.2 | 96.5 |
| Comparative Example 9 | 20 | 87.9 | 96.9 |

[Example 7]

The process up to the measurement of the cell information was performed in the same manner as in Example 6, except that the start time point t2 of the detachment operation was set to a time point at which the average brightness value reached 92.5. The start time point t2 of the detachment operation in this case was 7.3 minutes.

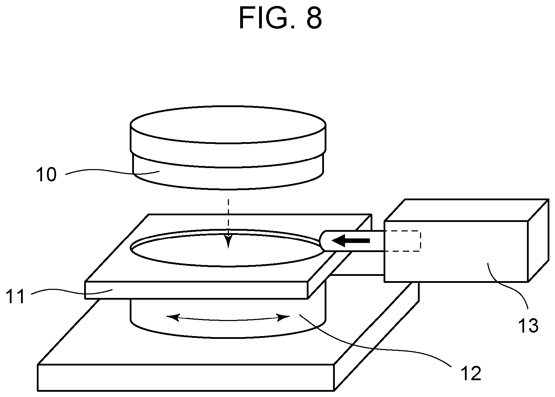

After the measurement of the cell information, a dish 10 was placed on a holding mechanism 11 by a shear stress application mechanism illustrated in Fig. 8. Using a rotation mechanism 12 and an impact application mechanism 13, a load of 5 N was applied to the outer periphery of the dish a total of 12 times at intervals of 5 seconds while the load application position was changed every 30° along the outer periphery of the dish. Thereafter, the cell survival rate and the cell detachment efficiency were evaluated in the same manner as in Example 1.

[Comparative Examples 10 to 13]

The cell survival rates and the cell detachment efficiencies were evaluated in the same manner as in Example 7, except that the cell information was not measured, and each start time point t2 was changed as given in Table 3.

Table 3 presents the results of the survival rates of the detached cells and the detachment efficiencies of the cells in Example 7 and Comparative Examples 10 to 13.

[Table 3]

Table 3

|  | Start time point t2 [min] | Survival rate of detached cells [%] | Detachment efficiency of cells [%] |
|---|---|---|---|
| Example 7 | 7.3 | 96.8 | 96.2 |
| Comparative Example 10 | 2 | 71.3 | 35.4 |
| Comparative Example 11 | 5 | 88.5 | 85.0 |
| Comparative Example 12 | 12 | 94.0 | 96.9 |
| Comparative Example 13 | 25 | 85.4 | 97.3 |

Embodiments of the present invention can also be provided by performing one or more functions of the above-described embodiments with a computer of a system or an apparatus (for example, an application-specific integrated circuit (ASIC)) that reads out and executes computer-executable instructions (for example, one or more programs) recorded on a storage medium, and/or by using a computer of a system or an apparatus that includes one or more circuits for executing one or more functions of the above-described embodiments, and by reading out and executing computer-executable instructions from a storage medium in order to perform, for example, one or more functions of the above-described embodiments, and/or by using a method performed by a computer of a system or an apparatus by controlling one or more circuits in order to perform one or more functions of the above-described embodiments. The computer can include one or more processors (for example, a central processing unit (CPU) and a micro-processing unit (MPU)) and can include a network of separate computers or separate processors to read out and execute computer-executable instructions. The computer-executable instructions may be provided to the computer, for example, from a network or the storage medium. The storage medium may include, for example, one or more of a hard disk, random-access memory (RAM), read-only memory (ROM), a storage device of distributed computing systems, an optical disk (for example, a compact disc (CD), digital versatile disc (DVD), or Blu-ray Disc (BD)), a flash memory device, a memory card, and so forth.

The disclosure of the embodiment includes the following methods and configurations.

(Method 1)

A cell detachment method for detaching a cell adhering to a culture surface of a cell culture container, including an adhesion strength reduction step of reducing adhesion strength of a cell by applying a stimulus to the cell while the cell remains adhered to a cell culture container, a measurement step of measuring the cell and acquiring cell information at at least two time points, a detachment step of detaching the cell from the culture surface, and a determination step of determining, based on the cell information, a time point $t2$ at which the detachment step is to be started, the time point $t2$ being after a time point $t1$, the time point $t1$ being a later time point among the at least two time points.

(Method 2)

The cell detachment method described in Method 1, in which the cell detachment method includes an incubation step of bringing the cell into contact with a cell detachment solution and incubating the cell.

(Method 3)

The cell detachment method described in Method 1 or 2, in which the time point $t1$ is a time point that is 30 seconds or more and 15 minutes or less from a start time point $t0$ of the adhesion strength reduction step.

(Method 4)

The cell detachment method described in any one of Methods 1 to 3, in which in the measurement step, an interval between the at least two time points among the time points at which the cell information is acquired is 1 second or more and 1 minute or less.

(Method 5)

The cell detachment method described in any one of Methods 1 to 4, in which a start time point $t0$ of the adhesion strength reduction step, the time point $t1$, and the time point $t2$ satisfy a relationship $(t2 - t0)/(t1 - t0) \geq 1.1$.

(Method 6)

The cell detachment method described in any one of Methods 1 to 5, in which the detachment step includes a step of subjecting the cell to vibration induced by ultrasound.

(Method 7)

The cell detachment method described in any one of Methods 1 to 6, in which in the determination step, a fitted curve is obtained based on the cell information and a time point at which the cell information has been acquired, and the time point $t2$ is determined based on the fitted curve.

(Method 8)

The cell detachment method described in Method 7, in which in the determination step, a time point at which the cell information calculated based on the fitted curve is equal to a predetermined threshold value of the cell information is determined as the time point $t2$.

(Method 9)

The cell detachment method described in Method 7, in which in the determination step, curve fitting is performed using the following expression as the fitted curve,
[Math. 9]

$$f(t) = C_0 + C_1\left(1 - \exp\left(-\left(\frac{t}{\tau}\right)^{\beta}\right)\right) \quad \cdots (2-1)$$

where t is a time point t at which the cell information has been acquired when a start time point of the adhesion strength reduction step is set to 0, f(t) is the cell information acquired at the time point t, A that is 0.3 or more and 2 or less is provided in advance, and a time point $A\tau$ that is A times $\tau$ is determined as the time point t2.

(Method 10)

The cell detachment method described in Method 7, in which in the determination step, curve fitting is performed using the following expression as the fitted curve,
[Math. 10]

$$f(t) = C_0 + C_1\left(1 - \exp\left(-\left(\frac{t}{\tau}\right)^{\beta}\right)\right) \quad \cdots (2-1)$$

where t is a time point t at which the cell information has been acquired when a start time point of the adhesion strength reduction step is set to 0, f(t) is the cell information acquired at the time point t, B that is 0.3 or more and 0.9 or less is provided in advance, and a time point at which f(t) is equal to $C_0 + C_1 \times B$ is determined as the time point t2.

(Method 11)

The cell detachment method described in any one of Methods 1 to 10, in which in the determination step, a time point t3 at which the detachment step is completed is determined.

(Method 12)

The cell detachment method described in any one of Methods 1 to 11, in which the cell information is measured from an image of the cell and is at least one of an area of the cell, a rate of change in the area of the cell, a brightness value of the cell, an area fraction of a region of the image having a brightness value more than a threshold value, and the brightness value of the image.

(Method 13)

The cell detachment method described in any one of Methods 1 to 12, in which the measurement step is performed after the adhesion strength reduction step is started.

(Method 14)

The cell detachment method described in any one of Methods 1 to 13, in which the fitted curve is a fitted curve of a time elapsed after a start of the adhesion strength reduction step and a brightness value of the cell.

(Method 15)

The cell detachment method described in any one of Methods 1 to 13, in which the fitted curve is a fitted curve of a time elapsed after a start of the adhesion strength reduction step and an area of the cell.

(Method 16)

A cell detachment method for detaching a cell adhering to a culture surface of a cell culture container, including an adhesion strength reduction step of reducing adhesion strength of a cell by applying a stimulus to the cell while the cell remains adhered to a cell culture container, a measurement step of measuring the cell and acquiring time-series data relating to the cell, a detachment step of detaching the cell from the culture surface, and a prediction step of predicting a time point at which the detachment step is to be started based on the time-series data.

(Method 17)

The cell detachment method described in Method 16, in which the time-series data is cell information measured at at least two time points and a time point at which the cell information has been measured, and the cell information is at least one of a brightness value of the cell and an area of the cell.

(Configuration 1)

A program for causing a computer to execute the cell detachment method described in any one of Methods 1 to 17.

(Configuration 2)

A cell detachment system for detaching a cell adhering to a culture surface of a cell culture container, including an adhesion strength reduction unit to reduce adhesion strength of a cell by applying a stimulus to the cell while the cell remains adhered to a cell culture container, a measurement unit to measure the cell and acquire cell information at at least two time points, a detachment unit to detach the cell from a culture surface, and a determination unit to determine, based on the cell information, a time point t2 at which a detachment step of detaching the cell from the culture surface is to be started, the time point t2 being after a time point t1, the time point t1 being a later time point among the at least two time points.

(Configuration 3)

A cell detachment system for detaching a cell adhering to a culture surface of a cell culture container, including an adhesion strength reduction unit to reduce adhesion strength of a cell by applying a stimulus to the cell while the cell remains adhered to a cell culture container, a measurement unit to measure the cell and acquire time-series data relating to the cell, a detachment unit to detach the cell from a culture surface, and a prediction unit to predict, based on the time-series data, a time point at which a detachment step of detaching the cell from the culture surface is to be started.

(Configuration 4)

An information processing device for controlling a cell detachment apparatus to detach a cell adhering to a culture surface of a cell culture container, the information processing device including a measurement unit to acquire, at at least two time points, cell information of a cell having reduced adhesion strength to a culture surface due to application of a stimulus, and a determination unit to determine, based on the cell information, a time point t2 at which the detachment step of detaching the cell from the culture surface is to be started, the time point t2 being after a time point t1, the time point t1 being a later time point among the at least two time points.

(Configuration 5)

An information processing device for controlling a cell detachment apparatus to detach a cell adhering to a culture surface of a cell culture container, the information processing device including a measurement unit to acquire, at at least two time points, time-series data relating to a cell having reduced adhesion strength to a culture surface due to application of a stimulus, and a prediction unit to predict, based on the time-series data, a time point at which a detachment step of detaching the cell from the culture surface is to be started.

The present invention is not limited to the above embodiments and various changes and modifications can be made within the spirit and scope of the present invention. Therefore, to apprise the public of the scope of the present invention, the following claims are made.

This application claims priority to Japanese Patent Application No. 2022-190040 filed November 29, 2022, which is hereby incorporated by reference herein in its entirety.

**Claims**

1. A cell detachment method for detaching a cell adhering to a culture surface of a cell culture container, comprising:

    an adhesion strength reduction step of reducing adhesion strength of a cell by applying a stimulus to the cell while the cell remains adhered to a cell culture container;

a measurement step of measuring the cell and acquiring cell information at at least two time points;

a detachment step of detaching the cell from the culture surface; and

a determination step of determining, based on the cell information, a time point t2 at which the detachment step is to be started, the time point t2 being after a time point t1, the time point t1 being a later time point among the at least two time points.

2. The cell detachment method according to Claim **1,**
wherein the cell detachment method includes an incubation step of bringing the cell into contact with a cell detachment solution and incubating the cell.

3. The cell detachment method according to Claim 1,
wherein the time point t1 is a time point that is 30 seconds or more and 15 minutes or less from a start time point t0 of the adhesion strength reduction step.

4. The cell detachment method according to Claim 1,
wherein in the measurement step, an interval between the at least two time points among the time points at which the cell information is acquired is 1 second or more and 1 minute or less.

5. The cell detachment method according to Claim 1,
wherein a start time point t0 of the adhesion strength reduction step, the time point t1, and the time point t2 satisfy a relationship (t2 - t0)/(t1 - t0) ≥ 1.1.

6. The cell detachment method according to Claim 1,
wherein the detachment step includes a step of subjecting the cell to vibration induced by ultrasound.

7. The cell detachment method according to Claim 1,
wherein in the determination step, a fitted curve is obtained based on the cell information and a time point at which the cell information has been acquired, and the time point t2 is determined based on the fitted curve.

8. The cell detachment method according to Claim 7, wherein in the determination step, a time point at which the cell information calculated based on the fitted curve is equal to a predetermined threshold value of the cell information is determined as the time point t2.

9. The cell detachment method according to Claim 7,

wherein in the determination step, curve fitting is performed using the following expression as the fitted curve,
[Math. 1]

$$f(t) = C_0 + C_1 \left( 1 - \exp\left(-\left(\frac{t}{\tau}\right)^\beta\right)\right) \quad \cdot \cdot \cdot (2-1)$$

where t is a time point t at which the cell information has been acquired when a start time point of the adhesion strength reduction step is set to 0, f(t) is the cell information acquired at the time point t, A that is 0.3 or more and 2 or less is provided in advance, and a time point Aτ that is A times τ is determined as the time point t2.

10. The cell detachment method according to Claim 7,

wherein in the determination step, curve fitting is performed using the following expression as the fitted curve,
[Math. 2]

$$f(t) = C_0 + C_1 \left( 1 - \exp\left(-\left(\frac{t}{\tau}\right)^\beta\right)\right) \quad \cdot \cdot \cdot (2-1)$$

where t is a time point t at which the cell information has been acquired when a start time point of the adhesion strength reduction step is set to 0, f(t) is the cell information acquired at the time point t, B that is 0.3 or more and 0.9 or less is provided in advance, and a time point at which f(t) is equal to $C_0 + C_1 \times B$ is determined as the time point t2.

11. The cell detachment method according to Claim 1,
wherein in the determination step, a time point t3 at which the detachment step is completed is determined.

12. The cell detachment method according to Claim 1,
wherein the cell information is measured from an image of the cell and is at least one of an area of the cell, a rate of change in the area of the cell, a brightness value of the cell, an area fraction of a region of the image having a brightness value more than a threshold value, and the brightness value of the image.

13. The cell detachment method according to Claim 1,
wherein the measurement step is performed after the adhesion strength reduction step is started.

14. The cell detachment method according to Claim 1,
wherein the fitted curve is a fitted curve of a time elapsed after a start of the adhesion strength reduction step and a brightness value of the cell.

15. The cell detachment method according to Claim 1,
wherein the fitted curve is a fitted curve of a time elapsed after a start of the adhesion strength reduction step and an area of the cell.

16. A cell detachment method for detaching a cell adhering to a culture surface of a cell culture container, comprising:

an adhesion strength reduction step of reducing adhesion strength of a cell by applying a stimulus to the cell while the cell remains adhered to a cell culture container;
a measurement step of measuring the cell and acquiring time-series data relating to the cell;
a detachment step of detaching the cell from the culture surface; and
a prediction step of predicting a time point at which the detachment step is to be started based on the time-series data.

17. The cell detachment method according to Claim 16,
wherein the time-series data is cell information measured at at least two time points and a time point at which the cell information has been measured, and
the cell information is at least one of a brightness value of the cell and an area of the cell.

18. A program for causing a computer to execute the cell detachment method according to any one of Claims 1 to 17.

19. A cell detachment system for detaching a cell adhering to a culture surface of a cell culture container, comprising:

an adhesion strength reduction unit to reduce adhesion strength of a cell by applying a stimulus to the cell while the cell remains adhered to a cell culture container;
a measurement unit to measure the cell and acquire cell information at at least two time points;
a detachment unit to detach the cell from a culture surface; and
a determination unit to determine, based on the cell information, a time point t2 at which a detachment step of detaching the cell from the culture surface is to be started, the time point t2 being after a time point t1, the time point t1 being a later time point among the at least two time points.

20. A cell detachment system for detaching a cell adhering to a culture surface of a cell culture container, comprising:

an adhesion strength reduction unit to reduce adhesion strength of a cell by applying a stimulus to the cell while the cell remains adhered to a cell culture container;
a measurement unit to measure the cell and acquire time-series data relating to the cell;
a detachment unit to detach the cell from a culture surface; and
a prediction unit to predict, based on the time-series data, a time point at which a detachment step of detaching the cell from the culture surface is to be started.

21. An information processing device for controlling a cell detachment apparatus to detach a cell adhering to a culture surface of a cell culture container, the information processing device comprising:

a measurement unit to acquire, at at least two time points, cell information of a cell having reduced adhesion strength to a culture surface due to application of a stimulus; and

a determination unit to determine, based on the cell information, a time point t2 at which the detachment step of detaching the cell from the culture surface is to be started, the time point t2 being after a time point t1, the time point t1 being a later time point among the at least two time points.

22. An information processing device for controlling a cell detachment apparatus to detach a cell adhering to a culture surface of a cell culture container, the information processing device comprising:

a measurement unit to acquire, at at least two time points, time-series data relating to a cell having reduced adhesion strength to a culture surface due to application of a stimulus; and

a prediction unit to predict, based on the time-series data, a time point at which a detachment step of detaching the cell from the culture surface is to be started.

# FIG. 1

# FIG. 2

EP 4 628 571 A1

# FIG. 3

| | INCUBATION TIME: 30 s | INCUBATION TIME: 5 min |
|---|---|---|
| PHASE-CONTRAST MICROSCOPE IMAGE | | |
| BINARIZED IMAGE | | |

# FIG. 4

# FIG. 5

# FIG. 6

# FIG. 7

FIG. 8

# FIG. 9

```
        ┌──────────────┐
        │    START     │
        └──────┬───────┘
               │
               ▼
   ┌───────────────────────────┐
   │  REDUCING CELL ADHESION    │──~ S101
   │ STRENGTH TO CULTURE SURFACE│
   └─────────────┬─────────────┘
                 │
                 ▼
   ┌───────────────────────────┐
   │  ACQUIRING CELL INFORMATION│──~ S102
   └─────────────┬─────────────┘
                 │
                 ▼
   ┌───────────────────────────┐
   │ DETERMINING TIME POINT AT WHICH│──~ S103
   │ DETACHMENT STEP IS TO BE STARTED│
   └─────────────┬─────────────┘
                 │
                 ▼
   ┌───────────────────────────┐
   │      DETACHING CELL        │──~ S104
   └─────────────┬─────────────┘
                 │
                 ▼
        ┌──────────────┐
        │     END      │
        └──────────────┘
```

# FIG. 10

```
        ┌─────────────┐
        │    START    │
        └─────────────┘
               │
               ▼
┌──────────────────────────────────┐
│  BRINGING CELL INTO CONTACT WITH CELL   │──── S201
│ DETACHMENT SOLUTION AND INCUBATING CELL │
└──────────────────────────────────┘
               │
               ▼
┌──────────────────────────────────┐
│    MEASURING CHANGE IN AVERAGE      │──── S202
│     BRIGHTNESS VALUE OVER TIME      │
└──────────────────────────────────┘
               │
               ▼
┌──────────────────────────────────┐
│       OBTAINING FITTED CURVE        │──── S203
└──────────────────────────────────┘
               │
               ▼
┌──────────────────────────────────┐
│   DETERMINING TIME POINT AT WHICH   │──── S204
│  DETACHMENT STEP IS TO BE STARTED   │
└──────────────────────────────────┘
               │
               ▼
┌──────────────────────────────────┐
│         DETACHING CELL BY           │──── S205
│      ULTRASONIC VIBRATION           │
└──────────────────────────────────┘
               │
               ▼
        ┌─────────────┐
        │     END     │
        └─────────────┘
```

# FIG. 11

| | |
|---|---|
| ADHESION STRENGTH REDUCTION UNIT | 201 |
| MEASUREMENT UNIT | 202 |
| DETERMINATION UNIT | 203 |
| DETACHMENT UNIT | 204 |

200

## INTERNATIONAL SEARCH REPORT

| International application No. |
|---|
| **PCT/JP2023/042136** |

| | | |
|---|---|---|
| **A.** | **CLASSIFICATION OF SUBJECT MATTER** | |

*C12N 5/07*(2010.01)i; *C12M 1/00*(2006.01)i; *C12N 1/00*(2006.01)i
FI:  C12N5/07; C12M1/00 A; C12N1/00 A

According to International Patent Classification (IPC) or to both national classification and IPC

| | |
|---|---|
| **B.** | **FIELDS SEARCHED** |

Minimum documentation searched (classification system followed by classification symbols)

C12N5/07; C12M1/00; C12N1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| Y | TAUCHI, H. et al. Effective and Intact Cell Detachment from a Clinically Ubiquitous Culture Flask by Combining Ultrasonic Wave Exposure and Diluted Trypsin. Biotechnology and Bioprocess Engineering. 2019, vol. 24, pp. 536-543<br>abstract, fig. 2 | 1-22 |
| Y | JP 2014-018185 A (TOKYO ELECTRON LIMITED) 03 February 2014 (2014-02-03)<br>abstract, claims, paragraphs [0006]-[0011], [0031] | 1-22 |
| Y | JP 2003-235540 A (TAYA, Masahito) 26 August 2003 (2003-08-26)<br>abstract, claims, paragraphs [0003]-[0017], fig. 1-7 | 1-22 |
| Y | WO 2007/136073 A1 (NIKON CORPORATION) 29 November 2007 (2007-11-29)<br>abstract, claims, paragraphs [0008]-[0026], fig. 1-8 | 1-22 |
| Y | 倉科佑太,竹村研治郎. 細胞培養への超音波振動技術の応用. 日本ロボット学会誌. 2018, vol. 36, no. 3, pp. 203-206, (KURASHINA, Yuta, TAKEMURA, Kenjiro. Application of Ultrasonic Vibration for Cell Culture. Journal of the Robotics Society of Japan.)<br>section 3.2, fig. 4 | 1-22 |

☑ Further documents are listed in the continuation of Box C. ☑ See patent family annex.

| | |
|---|---|
| * | Special categories of cited documents: |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **07 February 2024** | **20 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
| --- |
| **PCT/JP2023/042136** |

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2018-201403 A (NIPPON ZEON CO.) 27 December 2018 (2018-12-27) abstract, claims, paragraph [0041] | 1-22 |
| P, A | WO 2023/145797 A1 (CANON KABUSHIKI KAISHA) 03 August 2023 (2023-08-03) abstract, claims, paragraphs [0008]-[0013], fig. 1-7 | 1-22 |

Form PCT/ISA/210 (second sheet) (July 2022)

## INTERNATIONAL SEARCH REPORT
### Information on patent family members

International application No.

**PCT/JP2023/042136**

| Patent document cited in search report | | | Publication date (day/month/year) | Patent family member(s) | | | Publication date (day/month/year) |
|---|---|---|---|---|---|---|---|
| JP | 2014-018185 | A | 03 February 2014 | WO | 2014/017481 | A1 | |
| | | | | abstract, claims, paragraphs [0007]-[0012], [0032] | | | |
| JP | 2003-235540 | A | 26 August 2003 | (Family: none) | | | |
| WO | 2007/136073 | A1 | 29 November 2007 | US | 2009/0081769 | A1 | |
| | | | | abstract, claims, paragraphs [0036]-[0054], fig. 1-8 | | | |
| | | | | EP | 2022845 | A1 | |
| JP | 2018-201403 | A | 27 December 2018 | (Family: none) | | | |
| WO | 2023/145797 | A1 | 03 August 2023 | JP | 2023-111839 | A | |
| | | | | abstract, claims, paragraphs [0008]-[0013], fig. 1-7 | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003235540 A
- JP 2007136073 A
- JP 2004344049 A
- JP 2008092857 A
- JP 2006314204 A
- WO 2016047368 A
- JP 2022190040 A